# EUROPEAN PATENT SPECIFICATION

(11) **EP 2 585 028 B1**
(45) Date of publication and mention of the grant of the patent: **10.08.2016**
(21) Application number: 11729951.1
(22) Date of filing: 24.06.2011
(51) Int. Cl.: A61K 8/11, B01J 13/14, B01J 13/16, C11D 3/50

(54) **PROCESS FOR PRODUCING MICROCAPSULES**
VERFAHREN ZUR HERSTELLUNG VON MIKROKAPSELN
PROCÉDÉ DE PRODUCTION DE MICROCAPSULES

(30) Priority: 25.06.2010 GB 201010701
(43) Date of publication of application: 01.05.2013
(73) Proprietor: Givaudan SA, 1214 Vernier (CH)
(72) Inventor: HOTZ, Jutta, CH-8006 Zürich (CH); DENUELL, Wolfgang, 68163 Mannheim (DE)
(74) Representative: Givaudan Patents
(86) International application number: PCT/EP2011/060599
(87) International publication number: WO 2011/161229

(56) References cited:
- US-A- 5 011 885
- US-A- 5 342 556

## Description

The application relates to a process for producing microcapsules, and to uses of those microparticles in consumer products.

Microcapsules are powders or particles which consist of a core and a wall material surrounding the core, wherein the core is a solid, liquid or gaseous substance which is surrounded by the solid, generally polymeric, wall material. They may be solid, i.e. consist of a single material. Microcapsules have on average a diameter from 1 to 1000 µm.

A multitude of shell materials is known for producing microcapsules. The shell can consist either of natural, semisynthetic or synthetic materials. Natural shell materials are, for example, gum arabic, agar agar, agarose, maltodextrins, alginic acid or its salts, e.g. sodium alginate or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic shell materials are inter alia chemically modified celluloses, in particular cellulose esters and cellulose ethers, e.g. cellulose acetate, ethyl cellulose, hydroxypropylcellulose, hydroxypropylmethylcellulose and carboxymethylcellulose, and also starch derivatives, in particular starch ethers and starch esters. Synthetic shell materials are, for example, polymers such as polyacrylates, polyamides, polyvinyl alcohol or polyvinylpyrrolidone.

Depending on the type of shell material and the production process, microcapsules are formed in each case with different properties as far as diameter, size distribution and physical and/or chemical properties are concerned.

There is therefore a continuing need to develop novel production processes in order to be able to provide microcapsules with tailored properties.

A first subject matter of the present application is a consumer product as defined in claims.

A second subject matter of the application is the use of microcapsules as defined in the claims. The present application is also directed to a process for producing microcapsules which contain a shell and a fragrance oil core, where an aqueous solution of a protective colloid and a solution of a mixture of at least two structurally different at least difunctional isocyanates (A) and (B) in said oil are brought together until an emulsion is formed, to which an at least difunctional amine is then added, and which is then heated to temperatures of at least 60°C until the microcapsules are formed, wherein the isocyanate (B) is selected from the anionically modified isocyanates or from polyethylene oxide-containing isocyanates or mixtures of these types and the isocyanate (A) is uncharged, but is not a polyethylene-containing isocyanate.

The process has the advantage that microcapsules of a pre-given size or size distribution can be produced in a targeted manner, it being possible here to produce in particular relatively small microcapsules with diameters from 10 to 60 µm. Moreover, capsules with greater mechanical stability are obtained. Here, in particular those capsules are obtained, the shells of which have only a low permeability to the liquid ingredients.

In principle, an aqueous solution of the protective colloid is always produced, and for this the isocyanates (A) and (B) are dissolved in the fragrance oil, which later forms the core of the microcapsules; the amine components are then added and the mixture is heated until an emulsion is formed. The temperature for the reaction of the isocyanates with the amine components must be at least 60°C, but better 70°C, but preferably 75 to 90°C and in particular 85 to 90°C, in order to ensure sufficiently rapid reaction progress.

Here, it may be preferred to increase the temperature in stages (e.g. in each case by 10°C) until then, following completion of the reaction, the dispersion is cooled to room temperature (21°C). The reaction time typically depends on the amounts and temperatures used. Usually, however, the elevated temperature for forming the microcapsules is established between ca. 60 minutes to 6 h or up to 8 h.

According to the present teaching, the addition of the amine also preferably takes place with the input of energy, e.g. by using a stirring apparatus.

In order to form an emulsion in the present process, the respective mixtures are emulsified by processes known to the person skilled in the art, e.g. by introducing energy into the mixture through stirring using a suitable stirrer until the mixture emulsifies. The pH is preferably adjusted using aqueous bases, preference being given to using sodium hydroxide solution (e.g. 5% strength by weight).

It is essential to the process that at least two structurally different isocyanates (A) and (B) are used. These can be added in the form of a mixture or separately from one another in the process to the aqueous premix (1) containing the protective colloid and are then emulsified and reacted with the amine. It is also conceivable to meter in both mixtures of (A) and (B), and also the individual isocyanates (A) and (B) separately at different times.

In one preferred embodiment, the process is carried out as follows:
(a) a premix (I) is prepared from water and a protective colloid;
(b) this premix is adjusted to a pH in the range from 5 to 12;
(c) a further premix (II) is prepared from the fragrance oil together with the isocyanates (A) and (B);
(d) the two premixes (I) and (II) are brought together until an emulsion is formed and
(e) the at least difunctional amine is then metered into the emulsion from step (d) and
(f) the emulsion is then heated to temperatures of at least 60°C until the microcapsules are formed.

It may be advantageous to adjust the pH in step (b) to values from 8 to 12. Of suitability here are aqueous bases, preferably aqueous sodium hydroxide solution. The formation of the emulsion in step (d), but also step (e) is preferably ensured by using a suitable stirrer.

Another likewise preferred embodiment envisages that
(a) a premix (I) is prepared from water and a protective colloid;
(b) this premix is adjusted to a pH in the range from 5 to 12;
(c) a further premix (II) is prepared from a fragrance oil with the isocyanate (A);
(d) an emulsion is formed from the premixes (I) and (II) by stirring and to this
(e) is added the second isocyanate (B), and then the pH of the emulsion is adjusted to a value from 5 to 10;
(f) and then the at least difunctional amine is metered into the emulsion from step (e) and
(g) then heated to temperatures of at least 60°C until the microcapsules are formed.

In this procedure, the isocyanates (A) and (B) are added separately to the protective colloid before the addition of the amine and the reaction to give the microcapsules takes place. The formation of the emulsion - like the mixing in step (e) also takes place here preferably by using a stirring apparatus.

The pH in step (e) is preferably adjusted to values from 7.5 to 9.0. For step (b), the value can likewise be adjusted from 8 to 12. Of suitability for this purpose are in particular aqueous bases, preferably aqueous sodium hydroxide solution.

### Microcapsules

Within the context of the present teaching, the microcapsules have a shell made of a reaction product of at least two different, at least difunctional isocyanates with amines, preferably with polyamines. The reaction is a polycondensation between the isocyanates and the amines, which leads to a polyurea derivative.

The microcapsules may be present in the form of aqueous dispersions, the weight fraction of these dispersions in the capsules being preferably between 15 and 45% by weight and preferably 20 to 40% by weight. The microcapsules have an average diameter in the range from 1 to 500 µm and preferably from 1 to 50 µm or from 5 to 25 µm.

The amount of fragrance oil can vary in the range from 10 to 95% by weight, based on the weight of the capsules, where fractions from 70 to 90% by weight may be advantageous. As a result of the process, capsules are obtained which typically have core/shell ratios (w/w) from 20:1 to 1:10, preferably from 5:1 to 2:1 and in particular from 4:1 to 3:1.

The microcapsules which are produced by the present process are preferably free from formaldehyde.

### Protective colloid

During the reaction between the isocyanates and the amines, a protective colloid must be present. This is preferably a polyvinylpyrrolidone (PVP). Protective colloids are polymer systems which, in suspensions or dispersions, prevent a clumping together (agglomeration, coagulation, flocculation) of the emulsified, suspended or dispersed substances. During solvation, protective colloids bind large amounts of water and in aqueous solutions produce high viscosities depending on the concentration. Within the context of the process described herein, the protective colloid may also have emulsifying properties. The aqueous protective colloid solution is likewise preferably prepared with stirring.

The protective colloid may be, but does not have to be, a constituent of the capsule shell, with amounts from 0.1 to at most 15% by weight, but preferably in the range from 1 to 5% by weight and in particular from 1.5 to 3% by weight, based on the weight of the capsules, being possible here.

### Isocyanates

Isocyanates are N-substituted organic derivatives (R-N=C=O) of isocyanic acid (HNCO) tautomeric in the free state with cyanic acid. Organic isocyanates are compounds in which the isocyanate group (-N=C=O) is bonded to an organic radical. Polyfunctional isocyanates are those compounds with two or more isocyanate groups in the molecule.

According to the invention, at least difunctional, preferably polyfunctional, isocyanates are used, i.e. all aromatic, alicyclic and aliphatic isocyanates are suitable provided they have at least two reactive isocyanate groups.

The suitable polyfunctional isocyanates preferably contain on average 2 to at most 4 NCO groups. Preference is given to using diisocyanates, i.e. esters of isocyanic acid with the general structure O=C=N-R-N=C=O, where R' here is aliphatic, alicyclic or aromatic radicals.

Suitable isocyanates are, for example, 1,5-naphthylene diisocyanate, 4,4'-diphenylmethane diisocyanate (MOI), hydrogenated MDI (H12MDI), xylylene diisocyanate (XDI), tetramethylxylol diisocyanate (TMXDI), 4,4'-diphenyl-dimethylmethane diisocyanate, di- and tetraalkyldiphenylmethane diisocyanate, 4,4'-dibenzyl diisocyanate, 1,3-phenylene diisocyanate, 1,4-phenylene diisocyanate, the isomers of tolylene diisocyanate (TDI), optionally in a mixture, 1-methyl-2,4-diisocyanatocyclohexane, 1,6-diisocyanato-2,2,4-trimethylhexane, 1,6-diisocyanato-2,4,4-trimethylhexane, 1-isocyanatomethyl-3-isacyanato-1,5,5-trimethylcyclohexane, chlorinated and brominated diisocyanates, phosphorus-containing diisocyanates, 4,4'-diisocyanatophenylperfluoroethane, tetramethoxybutane 1,4-diisocyanate, butane 1,4-diisocyanate, hexane 1,6-diisocyanate (HDI), dicyclohexylmethane diisocyanate, cyclohexane 1,4-diisocyanate, ethylene diisocyanate, phthalic acid bisisocyanatoethyl ester, also polyisocyanates with reactive halogen atoms, such as 1-chloromethylphenyl 2,4-diisocyanate, 1-bromomethylphenyl 2,6-diisocyanate, 3,3-bischloromethyl ether 4,4'-diphenyldiisocyanate. Sulfur-containing polyisocyanates are obtained, for example, by reacting 2 mol of hexamethylene diisocyanate with 1 mol of thiodiglycol or dihydroxydihexyl sulfide. Further suitable diisocyanates are trimethylhexamethylene diisocyanate, 1,4-diisocyanatobutane, 1,2-diisocyanatododecane and dimer fatty acid diisocyanate.

One essential feature of the present process is the obligatory use of two structurally different isocyanates (A) and (B).

Suitable isocyanates of type (A) are at least difunctional compounds (i.e. compounds containing at least two isocyanate groups -N=C=O).

Typical representatives may be hexamethylene diisocyanate (HDI), or derivatives thereof, e.g. HDI biuret (commercially available e.g. as Desmodur N3200), HDI trimers (commercially available as Desmodur N3300) or else dicyclohexylmethane diisocyanates (commercially available as Desmodur W). Toluene 2,4-diisocyanate or diphenylmethane diisocyanate is likewise suitable.

The second isocyanate of type (B) is structurally different from the isocyanate of type (A) and specifically the isocyanate of type (B) must either be an anionically modified isocyanate or a polyethylene oxide-containing isocyanate (or any desired mixtures of these two isocyanate types).

The anionically modified isocyanates are known per se. Preferably, these isocyanates of type (B) contain at least two isocyanate groups in the molecule. One or more sulfonic acid radicals are preferably present as anionic groups. Preferably, isocyanates of type (B) are selected which are oligomers, in particular trimers, of hexane 1,6-diisocyanate (HDI). Commercial products of these anionically modified isocyanates are known, for example, under the brand Bayhydur (Bayer), e.g. Bayhydur XP.

Polyethylene oxide-containing isocyanates (with at least two isocyanate groups) are also known and are described, e.g. in US 5,342,556. Some of these isocyanates are self-emulsifying in water, which may be advantageous within the context of the present process since it may be possible to dispense with a separate emulsifying step.

The weight ratio of the two isocyanates (A) and (B) is adjusted in the range from 10:1 to 1:10, but in particular in the range from 5:1 to 1:5 and in particular in the range from 3:1 to 1:1.

It is also possible to use mixtures of different isocyanates of types (A) and (B). Besides the isocyanates (A) and (B), further isocyanates can also additionally be used in the process according to the invention.

Preferably, however, only anionically modified isocyanates are used as component (B) in the present process. Amines

At least difunctional amines, but preferably polyethyleneimines (PEI), are used as further component in the process according to the invention. Polyethyleneimines are generally polymers in the main chains of which there are NH groups which are separated from one another in each case by two methylene groups:

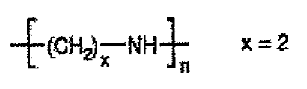

Polyethyleneimines belong to the polyelectrolytes and the complexing polymers. Short-chain, linear polyethyleneimines with a correspondingly high fraction of primary amino groups, i.e. products of the general formula H₂N [CH₂-CH₂-NH]*ₙ*H (*n* = 2: diethylenetriamine; *n* = 3; triethylenetetramine; *n* = 4: tetraethylenepentamine) are sometimes called polyethyleneamines or polyalkylenepolyamines.

In the processes according to the invention, polyethyleneimines with a molecular weight of at least 500 g/mol, preferably from 600 to 30 000 or 650 to 25 000 g/mol and in particular from 700 to 5000 g/mol or 850 to 2500 g/mol, are preferably used.

### Protective colloids

In the process according to the invention, PVP is used as protective colloid. PVP is the abbreviation for polyvinylpyrrolidones (also known as polyvidone). According to Römpp Chemie Lexikon, Online-edition 3.6, 2010, they are [poly(1-vinylpyrrolidin-2-ones)], i.e. polymers (vinyl polymers) which conform to the general formula:

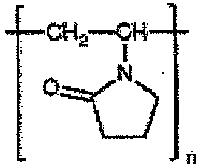

Standard commercial polyvinylpyrrolidones have molar masses in the range from ca. 2500-750 000 g/mol which are characterized by stating the K values and have - depending on the K value - glass transition temperatures from 130 to 175°C. They are supplied as white, hygroscopic powders or as aqueous solution.

In the processes according to the invention, preference is given to using PVPs with a high molecular weight, i.e. more than 400 000 g/mol and preferably from 500 000 g/mol to 2 000 000 g/mol. It is furthermore preferred for the polyvinylpyrrolidones to have a K value of more than 60, preferably more than 75 and in particular more than 80. A preferred range is between 65 and 90 for the K value.

### Fragrance Oil

The microcapsules produced using the process described above contain a fragrance oil core. The isocyanates should be soluble in the oil forming the core The term "fragrance oil" denotes one or a mixture of perfume components, optionally mixed with a suitable solvent, diluent, carrier or other adjuvant, which is intended to be used to impart a desired odour to a consumer product.

All manner of perfume ingredients may employed as will be clear to a person skilled in the art and it is not necessary to provide an exhaustive list here. Exemplary of perfume components and mixtures thereof which can be used for the preparation of such fragrance oils may include natural products such as essential oils, absolutes, resinoids, resins, concretes, etc., and synthetic perfume components such as hydrocarbons, alcohols, aldehydes, ketones, ethers, acids, esters, acetals, ketals, nitriles, etc., including saturated and unsaturated compounds, aliphatic, carbocyclic and heterocyclic compounds. Examples of such perfume components are: geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, benzyl benzoate, styrallyl acetate, amyl salicylate, dimethylbenzylcarbinol, trichloromethylphenycarbinyl acetate, p-tert.butylcyclohexyl acetate, isononyl acetate, vetiveryl acetate, vetiverol, alpha-n-amylcinammic aldehyde, alpha-hexylcinammic aldehyde, 2-methyl-3-(p-tert.butylphenyl)propanal, 2-methyl-3-(p-isopropylphenyl)propanal, 3-(p-tert.butylphenyl)propanal, tricyclodecenyl acetate, tricyclodecenyl propionate, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, methyl dihydrojasmonate, 2-n-heptylcyclopentanone, 3-methyl-2-pentylcyclopentanone, n-decanal, n-dodecanal,

9-decenol-1, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepine nitrile, aubepine, heliotropine, coumarin, eugenol, vanillin, diphenyl oxide, hydroxycitronellal, ionones, methyl ionones, isomethyl ionones, irones, cis-3-hexenol and esters thereof, indane musk fragrances, tetralin musk fragrances, isochroman musk fragrances, macrocyclic ketones, macrolactone musk fragrances, ethylene brassylate, aromatic nitromusk fragrances. The fragrance oils may also contain precursor or pro-fragrances of any perfume ingredients including any of those mentioned specifically above.

Suitable solvents, diluents or carriers for perfumes as mentioned above are for example: ethanol, isopropanol, diethylene glycol monoethyl ether, dipropylene glycol, diethyl phthalate, triethyl citrate and the like. Examples of carrier materials, diluents, solvents and other auxiliary agents commonly used in conjunction with fragrance oils can be found in, for example, in S. Arctander, 'Perfume and Flavour Materials of Natural Origin', Elizabeth, N.J., 1960, S. Arctander, 'Perfume and Flavour Chemicals', Vol. I and II, Allured Publishing Corporation, Carol Stream, 1994, and J.M. Nikitakis (Ed.), 'CTFA Cosmetic Ingredient Handbook', 1st ed., The Cosmetic, Toiletry and Fragrance Association, Inc., Washington, 1988.

The invention further provides aqueous dispersions comprising 5 to 50% by weight, based on the total weight of the dispersion, preferably from 15 to 40% by weight, of microcapsules which can be produced by the above process. A further preferred range is between 20 and 35% by weight. These aqueous dispersions are preferably obtained directly from the process described above.

The microcapsule dispersions which are obtained by the present process can be used to perfume all manner of consumer products. An exhaustive list of consumer products cannot be given here and the skilled person would appreciate the scope of application for such microcapsules. The consumer products are selected from the group consisting of laundry applications including softeners, liquid detergents, and powder detergents; personal care and hair care applications including shampoo, conditioners, combing creams, leave on conditioners, styling cream, soaps, body creams, deodorants and anti-perspirants; and household cleaning applications.

The present invention further provides preferably formaldehyde-free microcapsules containing a fragrance oil core, and a shell of a reaction product of at least two different at least difunctional isocyanates (A) and (B), where the isocyanate (B) must be an anionically modified isocyanate or a polyethylene oxide containing isocyanate or mixtures of the types, and an at least difunctional amine, with the proviso that during the production of the microcapsules the weight ratio between the isocyanates (A) and (B) is in the range from 10:1 to 1:10. Preferably, the aforementioned weight ratios can be adjusted, where the ratio from 3:1 to 1:1 may be attributed particular importance.

These microcapsules preferably have diameters from 1 to 50 µm and preferably diameters from 2 to 45 µm. They may be present in the form of an aqueous dispersion, where the fraction of the capsules can be 1 to 90% by weight, but preferably 5 to 50% by weight.

There now follows a series of examples that serve to illustrate the invention.

### Example 1 - Encapsulation

An oil phase was prepared when Desmodur W (Bayer) and Bayhydur XP2547 (Bayer) were added in perfume oil at a level of 12.6% and 3.4% respectively.

An aqueous phase (Solution S1) was prepared by adding Luviskol k90 (BASF) to water, at a level of 4.5%. The pH of the solution was adjusted at 10 by addition of a buffer pH=10 at 0.5%.

An aqueous phase (Solution S2) was prepared by adding Lupasol PR8515 (BASF) to water, at a level of 20%.

Capsules were prepared according to the following procedure:
300g of the oil phase was mixed with 600g of solution S1, to form an oil-in-water emulsion, in a 1 L reactor equipped with a MIG stirrer operating at 1000rpm.After 30 minutes of mixing, 100g of solution S2 was added over a period of 1 minute.After 30 minutes, the slurry was heated up to 70°C (1 H), then kept for 2H at 70°C, then heated to 80°C and kept for 1 H at 80°C, then heated to 85°C and kept for 1 H at 85°C, then cooled to 70°C and kept for 1 H at 70°C before final cooling at 25°C.

### Example 2 - Hair Care application

Hair Switch testing was carried out using standard hair protocols with a dosage of perfume of 0.2%. The capsules were prepared according to recipe given in example 1. The perfume composition is given in Table below. The performance of the capsules was evaluated by a direct comparison with the free perfume (non encapsulated oil).

| | % |
|---|---|
| AGRUMEX | 30 |
| AMYL BUTYRATE | 2.5 |
| GALBANONE | 10 |
| ETHYL 2 METHYL BUTYRATE | 2.5 |
| HEXYL ACETATE | 5 |
| NECTARYL | 5 |
| PECHE PURE | 10 |
| PRENYL ACETATE | 6 |
| TRIPLAL | 4 |
| VERDYL ACETATE | 25 |

### Protocol for shampoo

- Switches used: European hair, virgin, not damaged (but re-used several times)
- Dampen switch with warm water and place on weighing balance
- Squeeze 2.5 g of shampoo along the switch using a syringe
- Massage the shampoo into the hair switch for 30 seconds
- Leave the lathered switch to soak for 1 minute before rinsing out under running hand-hot water for approx. 30 seconds
- Squeeze the switch between two fingers to remove excess water
- Dry switch; either hang up to air dry or immediately blow dry using a hair dryer
- Leave air dried samples hanging in an odour free room for 24 hours
- Assess each switch before and after combing by use of a ten point scale: 0 = No odour, 9 = very strong

### Protocol for Hair conditioner:

The same protocol was followed for conditioner except the hair switches are prewashed in unfragranced shampoo before the conditioner is applied

| Sample | Performance in shampoo (before / after combing) | Performance in conditioner (before / after combing) |
|---|---|---|
| Free oil | 0.2/0.2 | 0.5/0.5 |
| capsule | 2.2/3.9 | 3.8/6.1 |

### Example 3 - Fabric Care Application

The capsules were prepared according to recipe given in Example 1. The perfume composition is given in Table below. The performance of the capsules was evaluated by a direct comparison with the free perfume (non encapsulated oil), on freshly prepared samples and after 1 month storage at 37°C.

| | % |
|---|---|
| AGRUMEX | 30 |
| AMYL BUTYRATE | 2.5 |
| GALBANONE | 10 |
| ETHYL 2 METHYL BUTYRATE | 2.5 |
| HEXYL ACETATE | 5 |
| NECTARYL | 5 |
| PECHE PURE | 10 |
| PRENYL ACETATE | 6 |
| TRIPLAL | 4 |
| VERDYL ACETATE | 25 |

### Protocol for Fabric detergent application

Washing conditions: 100g of perfumed powder detergent, 1 kg of cotton towels, European washing machine. The perfumed samples were prepared at a level of 0.5% perfume in a standard powder detergent base and the washing conditions used were as follows:
- total weight of the wash was 1 kg
- European machines
- Assessment is done before and after rubbing, on line dried and tumble dried towels, by use of a 5 point scale: 0 = no odour; 5 = very strong

### Protocol for Fabric softener application

The perfumed samples were prepared at a level of 0.5% perfume in a standard fabric conditioner base comprising 13% Quaternium ammonium ARQUAD 2HT75 from Akzo, 0.3% Silicone Dow Corning DB110 from Dow Corning, 0.6% CaCl2 from Merck and 0.15% Bronidox from Henkel and the washing conditions used were as follow:
- total weight of the wash was 0.2kg
- wash with unperfumed laundry powder (90g of standard internal Givaudan laundry powder) done before adding 35g of the perfumed fabric conditioner
- European machines
- Assessment is done before and after rubbing, on line dried and tumble dried towels, by use of a 5 point scale: 0 = no odour; 5 = very strong

| Performance in powder detergent (before / after rubbing) | | | | |
|---|---|---|---|---|
| Sample | Freshly prepared | | After 1 month storage at 37°C | |
| | Line dried | Tumble dried | Line dried | Tumble dried |
| Free oil | 1/1 | 0.5/0.5 | 0.5/0.5 | 0.5/0.5 |
| capsule | 2.5/3.5 | 3/3.5 | 1/3 | 3/4 |

| Performance in Fabric conditioner (before / after rubbing) | | | | |
|---|---|---|---|---|
| Sample | Freshly prepared | | After 1 month storage at 37°C | |
| | Line dried | Tumble dried | Line dried | Tumble dried |
| Free oil | 1.5/1.5 | 1/1 | 1/1 | 0.5/0.5 |
| capsule | 2/3.5 | 2/3 | 1/3.5 | 1.5/3.5 |

## Claims

1. A consumer product selected from the group consisting of laundry applications including softeners, liquid detergents, and powder detergents; personal care and hair care applications including shampoo, conditioners, combing creams, leave on conditioners, styling cream, soaps, body creams; deodorants and anti-perspirants; and household cleaning applications,containing microcapsules comprising a fragrance oil core, and a shell of a reaction product of at least two different at least difunctional isocyanates (A) and (B), where the isocyanate (B) must be an anionically modified isocyanate or a polyethylene oxide-containing isocyanate or mixtures of these types, and an at least difunctional amine, with the proviso that during the production of the microcapsules the weight ratio between the isocyanates (A) and (B) is in the range from 10:1 to 1:10.

2. A consumer product according to claim 1 wherein the microcapsule has a diameter from 1 to 50 µm.

3. A consumer product according to claim 1 or claim 2 wherein microcapsule is present in the form of an aqueous dispersion.

4. A consumer product according to any of the preceding claims wherein the microcapsules are produced by a process wherein an aqueous solution of a protective colloid and a solution of a mixture of at least two structurally different at least difunctional diisocyanates (A) and (B) in a fragrance oil are brought together until an emulsion is formed, to which an at least difunctional amine is then added and which is then heated to temperatures of at least 60°C until the microcapsules are formed, wherein the isocyanate (B) is selected from the anionically modified isocyanates or the polyethylene oxide-containing isocyanates and the isocyanate (A) is uncharged and is not a polyethylene oxide-containing isocyanate.

5. A consumer product according to claim 4 wherein a polyvinylpyrrolidone is used as protective colloid.

6. A consumer product according to claim 4 or claim 5 wherein the isocyanate (A) is selected from the group consisting of hexane 1,6-diisocyanate, hexane 1,6-diisocyanate biuret or oligomers of hexane 1,6-diisocyanate, in particular trimers thereof or dicyclohexanemethylene diisocyanate.

7. A consumer product according to any of the claims 4 to 6 wherein the isocyanate (B) is selected from the group of anionically modified diisocyanates which contain at least one sulfonic acid group, preferably an aminosulfonic acid group, in the molecule.

8. A consumer product according to any of the claims 4 to 7 wherein the at least difunctional amine used is a polyethyleneimine.

9. A consumer product according to any of the claims 4 to 8 wherein the weight ratio between the isocyanates (A) and (B) is in the range from 10:1 to 1:10, preferably 5:1 to 1:5 and in particular from 3:1 to 1:1.

10. A consumer product according to any of the claims 4 to 9 wherein the core-shell ratio (w/w) of the microcapsules is 20:1 to 1:10, preferably 5:1 to 2:1 and in particular 4:1 to 3:1.

11. A consumer product according to any of the claims 4 to 10 wherein the process of forming the microcapsules proceeds according to the following steps:
(a) a premix (I) is prepared from water and a protective colloid;
(b) this premix is adjusted to a pH in the range from 5 to 12;
(c) a further premix (II) is prepared from the fragrance oil together with the isocyanates (A) and (B);
(d) the two premixes (I) and (II) are brought together until an emulsion is formed and
(e) the at least difunctional amine is then metered into the emulsion from step (d) and
(f) the emulsion is then heated to temperatures of at least 60°C until the microcapsules are formed.

12. A consumer product according to claim 11 wherein the pH in process step (b) is adjusted to 8 to 12.

13. The use of microcapsules as defined in any of the claims 1 to 12 for perfuming a consumer product selected from the group consisting of laundry applications including softeners, liquid detergents, and powder detergents; personal care and hair care applications including shampoo, conditioners, combing creams, leave on conditioners, styling cream, soaps, body creams; deodorants and anti-perspirants; and household cleaning applications.

## Patentansprüche

1. Verbraucherprodukt, ausgewählt aus der Gruppe bestehend aus Wäschereiapplikationen einschließlich Weichspülern, Flüssigreinigern und pulverförmigen Reinigern; Körperhygiene- und Haarpflegeapplikationen einschließlich Shampoo, Conditionern, Kämmcremes, Leave-On-Conditionern, Stylingcreme, Seifen, Körpercremes; Deodorantien und Antiperspirantien; und Haushaltsreinigungsapplikationen, enthaltend Mikrokapseln umfassend einen Duftölkern und eine Hülle aus einem Reaktionsprodukt aus mindestens zwei unterschiedlichen mindestens difunktionellen Isocyanaten (A) und (B), wobei das Isocyanat (B) ein anionisch modifiziertes Isocyanat oder ein polyethylenoxidhaltiges Isocyanat oder Mischungen dieser Typen sein muss, und mindestens einem difunktionellen Amin, mit der Maßgabe, dass das Gewichtsverhältnis zwischen den Isocyanaten (A) und (B) während der Herstellung der Mikrokapseln im Bereich von 10:1 bis 1:10 liegt.

2. Verbraucherprodukt nach Anspruch 1, wobei die Mikrokapsel einen Durchmesser von 1 bis 50 µm aufweist.

3. Verbraucherprodukt nach Anspruch 1 oder Anspruch 2, wobei die Mikrokapsel in Form einer wässrigen Dispersion vorliegt.

4. Verbraucherprodukt nach einem der vorhergehenden Ansprüche, wobei die Mikrokapseln nach einem Verfahren hergestellt werden, bei dem man eine wässrige Lösung eines Schutzkolloids und eine Lösung einer Mischung aus mindestens zwei strukturell unterschiedlichen mindestens difunktionellen Diisocyanaten (A) und (B) in einem Duftöl zusammenbringt, bis eine Emulsion entsteht, zu der dann ein mindestens difunktionelles Amin zugefügt wird und die dann auf Temperaturen von mindestens 60°C erhitzt wird, bis sich Mikrokapseln bilden, wobei das Isocyanat (B) aus anionisch modifizierten Isocyanaten oder den polyethylenoxidhaltigen Isocyanaten ausgewählt ist und das Isocyanat (A) ungeladen ist und kein polyethylenoxidhaltiges Isocyanat ist.

5. Verbraucherprodukt nach Anspruch 4, wobei ein Polyvinylpyrrolidon als Schutzkolloid verwendet wird.

6. Verbraucherprodukt nach Anspruch 4 oder Anspruch 5, wobei das Isocyanat (A) aus der Gruppe bestehend aus Hexan-1,6-diisocyanat, Hexan-1,6-diisocyanatbiuret oder Oligomeren von Hexan-1,6-diisocyanat, insbesondere Trimeren davon, oder Dicyclohexanmethylendiisocyanat ausgewählt ist.

7. Verbraucherprodukt nach einem der Ansprüche 4 bis 6, wobei das Isocyanat (B) aus der Gruppe anionisch modifizierter Diisocyanate, die mindestens eine Sulfonsäuregruppe, vorzugsweise eine Aminosulfonsäuregruppe, in dem Molekül enthalten, ausgewählt ist.

8. Verbraucherprodukt nach einem der Ansprüche 4 bis 7, wobei das verwendete mindestens difunktionelle Amin ein Polyethylenimin ist.

9. Verbraucherprodukt nach einem der Ansprüche 4 bis 8, wobei das Gewichtsverhältnis zwischen den Isocyanaten (A) und (B) im Bereich von 10:1 bis 1:10, vorzugsweise 5:1 bis 1:5 und insbesondere von 3:1 bis 1:1, liegt.

10. Verbraucherprodukt nach einem der Ansprüche 4 bis 9, wobei das Kern-Hülle-Verhältnis (w/w) der Mikrokapseln 20:1 bis 1:10, vorzugsweise 5:1 bis 2:1 und insbesondere 4:1 bis 3:1, beträgt.

11. Verbraucherprodukt nach einem der Ansprüche 4 bis 10, wobei das Verfahren der Mikrokapselbildung gemäß den folgenden Schritten abläuft:
(a) aus Wasser und einem Schutzkolloid wird eine Vormischung (I) hergestellt;
(b) diese Vormischung wird auf einen pH-Wert im Bereich von 5 bis 12 eingestellt;
(c) aus dem Duftöl zusammen mit den Isocyanaten (A) und (B) wird eine weitere Vormischung (II) hergestellt;
(d) die zwei Vormischungen (I) und (II) werden zusammengebracht, bis eine Emulsion entsteht, und
(e) das mindestens difunktionelle Amin wird dann in die Emulsion aus Schritt (d) eindosiert, und
(f) die Emulsion wird dann auf Temperaturen von mindestens 60°C erhitzt, bis die Mikrokapseln entstehen.

12. Verbraucherprodukt nach Anspruch 11, wobei der pH-Wert im Verfahrensschritt (b) auf 8 bis 12 eingestellt wird.

13. Verwendung von Mikrokapseln wie in einem der Ansprüche 1 bis 12 definiert zum Parfümieren eines Verbraucherprodukts, ausgewählt aus der Gruppe bestehend aus Wäschereiapplikationen einschließlich Weichspülern, Flüssigreinigern und pulverförmigen Reinigern; Körperhygiene- und Haarpflegeapplikationen einschließlich Shampoo, Conditionern, Kämmcremes, Leave-On-Conditionern, Stylingcreme, Seifen, Körpercremes; Deodorantien und Antiperspirantien; und Haushaltsreinigungsapplikationen.

## Revendications

1. Produit de consommation choisi dans le groupe constitué d'applications de blanchisserie comprenant des assouplissants, des détergents liquides, et des détergents en poudre ; des applications de soin personnel et de soin capillaire comprenant un shampoing, des conditionneurs, des crèmes de peignage, des conditionneurs sans rinçage, une crème de coiffure, des savons, des crèmes pour le corps ; des déodorants et des désodorisants ; et des applications de nettoyage domestique, contenant des microcapsules comprenant un noyau d'huile parfumée, et une enveloppe d'un produit de réaction d'au moins deux isocyanates au moins difonctionnels différents (A) et (B), où l'isocyanate (B) doit être un isocyanate anioniquement modifié ou un isocyanate contenant du poly(oxyde d'éthylène) ou des mélanges de ces types, et une amine au moins difonctionnelle, à condition que, pendant la production des microcapsules, le rapport en poids entre les isocyanates (A) et (B) soit dans la plage de 10:1 à 1:10.

2. Produit de consommation selon la revendication 1 dans lequel la microcapsule a un diamètre de 1 à 50 µm.

3. Produit de consommation selon la revendication 1 ou la revendication 2 dans lequel la microcapsule est présente sous la forme d'une dispersion aqueuse.

4. Produit de consommation selon l'une quelconque des revendications précédentes dans lequel les microcapsules sont produites par un procédé dans lequel une solution aqueuse d'un colloïde protecteur et une solution d'un mélange d'au moins deux diisocyanates au moins difonctionnels structuralement différents (A) et (B) dans une huile de parfum sont combinés conjointement jusqu'à ce qu'une émulsion soit formée, à laquelle une amine au moins difonctionnelle est ensuite ajoutée et qui est ensuite chauffée à des températures d'au moins 60 °C jusqu'à ce que les microcapsules soient formées, dans lequel l'isocyanate (B) est choisi parmi les isocyanates anioniquement modifiés ou les isocyanates contenant du poly(oxyde d'éthylène) et l'isocyanate (A) est non chargé et n'est pas un isocyanate contenant du poly(oxyde d'éthylène).

5. Produit de consommation selon la revendication 4 dans lequel une polyvinylpyrrolidone est utilisée en tant que colloïde protecteur.

6. Produit de consommation selon la revendication 4 ou la revendication 5 dans lequel l'isocyanate (A) est choisi dans le groupe constitué des 1,6-diisocyanate d'hexane, 1,6-diisocyanate d'hexane-biuret ou des oligomères de 1,6-diisocyanate d'hexane, en particulier des trimères de celui-ci ou le diisocyanate de dicyclohexaneméthylène.

7. Produit de consommation selon l'une quelconque des revendications 4 à 6 dans lequel l'isocyanate (B) est choisi dans le groupe de diisocyanates anioniquement modifiés qui contiennent au moins un groupe acide sulfonique, de préférence un groupe acide aminosulfonique, dans la molécule.

8. Produit de consommation selon l'une quelconque des revendications 4 à 7 dans lequel l'amine au moins difonctionnelle utilisée est une polyéthylénimine.

9. Produit de consommation selon l'une quelconque des revendications 4 à 8 dans lequel le rapport en poids entre les isocyanates (A) et (B) est dans la plage de 10:1 à 1:10, de préférence 5:1 à 1:5 et en particulier de 3:1 à 1:1.

10. Produit de consommation selon l'une quelconque des revendications 4 à 9 dans lequel le rapport noyau-enveloppe (m/m) des microcapsules est de 20:1 à 1:10, de préférence de 5:1 à 2:1 et en particulier de 4:1 à 3:1.

11. Produit de consommation selon l'une quelconque des revendications 4 à 10, le procédé de formation des microcapsules étant conduit selon les étapes suivantes :
(a) un prémélange (I) est préparé à partir d'eau et d'un colloïde protecteur ;
(b) ce prémélange est ajusté à un pH dans la plage de 5 à 12 ;
(c) un prémélange supplémentaire (II) est préparé à partir de l'huile de parfum conjointement avec les isocyanates (A) et (B) ;
(d) les deux prémélanges (I) et (II) sont combinés conjointement jusqu'à ce qu'une émulsion soit formée et
(e) l'amine au moins difonctionnelle est ensuite dosée dans l'émulsion de l'étape (d) et
(f) l'émulsion est ensuite chauffée à des températures d'au moins 60 °C jusqu'à ce que les microcapsules soient formées.

12. Produit de consommation selon la revendication 11, le pH dans l'étape de procédé (b) étant ajusté à 8 à 12.

13. Utilisation de microcapsules telles que définies dans l'une quelconque des revendications 1 à 12 pour parfumer un produit de consommation choisi dans le groupe constitué d'applications de blanchisserie comprenant des assouplissants, des détergents liquides, et des détergents en poudre ; des applications de soin personnel et de soin capillaire comprenant un shampoing, des conditionneurs, des crèmes de peignage, des conditionneurs sans rinçage, une crème de coiffure, des savons, des crèmes pour le corps ; des déodorants et des désodorisants ; et des applications de nettoyage domestique.
